(19) Europäisches Patentamt
European Patent Office
Office européen des brevets

(11) Veröffentlichungsnummer: **0 150 499**
**A2**

(12) # EUROPÄISCHE PATENTANMELDUNG

(21) Anmeldenummer: **84116315.7**

(22) Anmeldetag: **27.12.84**

(51) Int. Cl.[4]: **C 07 D 249/08**, C 07 D 233/60, A 01 N 43/653, A 01 N 43/50

(30) Priorität: **12.01.84 JP 2683/84**

(43) Veröffentlichungstag der Anmeldung: **07.08.85 Patentblatt 85/32**

(84) Benannte Vertragsstaaten: **AT BE CH DE FR GB IT LI NL**

(71) Anmelder: **NIHON TOKUSHU NOYAKU SEIZO K.K., No.4, 2-chome, Nihonbashi Honcho Chuo-ku, Tokyo 103 (JP)**

(72) Erfinder: **Kurahashi, Yoshio, 47-15, Ohya-machi Hachioji-shi, Tokyo (JP)**
Erfinder: **Matsumoto, Noboru, 39-15, Namiki-cho Hachioji-shi, Tokyo (JP)**
Erfinder: **Shiokawa, Kozo, 210-6, Shukugawara Tama-ku Kawasaki-shi, Kanagawa-ken (JP)**
Erfinder: **Kagabu, Shinzo, 432-131-105, Terada-machi Hachioji-shi, Tokyo (JP)**
Erfinder: **Moriya, Koichi, 39-15, Namiki-cho Hachioji-shi, Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

(54) **3-Substituierte Azolyl-butan-Derivate, Verfahren zu ihrer Herstellung und Fungizide für Landwirtschaft und Gartenbau.**

(57) Die vorliegende Erfindung betrifft neue 3-substituierte Azolyl-butan-Derivate, Verfahren zu ihrer Herstellung und ihre Verwendung als Fungizide in Landwirtschaft und Gartenbau.

Die Verbindungen der Formel

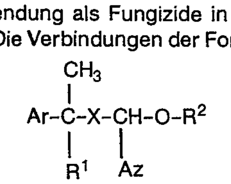

(I)

in der Ar, $R^1$, X, Az und $R^2$ die in der Beschreibung angegebenen Bedeutungen haben, wurden durch Reaktion eines 3-substituierten Halogeno-butan-Derivats mit einer Azol-Verbindung hergestellt. Einige spezielle Varianten sind in der Beschreibung erläutert.

Die Verbindungen der Formel (I) zeigen hohe Aktivität gegenüber phytopathogenen Pilzen, insbesondere pulvrigen Mehltau erzeugenden Pilzen.

EP 0 150 499 A2

# 3-Substituierte Azolyl-butan-Derivate, Verfahren zu ihrer Herstellung und Fungizide für Landwirtschaft und Gartenbau

Die vorliegende Erfindung betrifft neue 3-substituierte Azolyl-butan-Derivate, Verfahren zu ihrer Herstellung und ihre Verwendung als Fungizide in Landwirtschaft und Gartenbau.

Gemäß dem Stand der Technik besitzen einige 1-Aryloxy-1-(1,2,4-triazol-1-yl)-alkan-2-on-Derivate fungizide Aktivität (JP-OS 80738/1973, entsprechend der DE-OS 22 01 063 /‾Le A 14 118_7). Die betreffenden Beschreibungen offenbaren auch mögliche Aralkyl-Substituenten, empfehlen jedoch nur solche Aralkyl-Gruppen, die nicht der vorliegenden Erfindung entsprechende Aralkyl-Gruppen umfassen.

Weiterhin wurde beschrieben, daß einige 1-Aryloxy-1-imidazolyl-alkan-2-on-Derivate fungizide Aktivität besitzen (JP-OS 18633/1975, entsprechend der DE-OS 23 25 156 /‾Le A 14 999_7). Auch in diesem Fall sind Aralkyl-Gruppen als mögliche Substituenten aufgeführt, jedoch finden sich spezielle Angaben oder beispielhafte Nennungen zu den Aralkyl-Gruppen nicht.

Gleichfalls in Patent-Dokumenten erwähnt sind Triazolyl-O,N-acetal-Derivate und deren Salze mit fungizider Aktivität (JP-OS 13534/1975, entsprechend der DE-OS 23 24 010 [Le A 14 971]). Auch diese Schriften offenbaren mögliche Aralkyl-Substituenten, schließen jedoch ebenfalls die Aralkyl-Gruppen gemäß der vorliegenden Erfindung nicht ein.

Schließlich ist auch beschrieben, daß einige Imidazolyl-O,N-acetale oder deren Salze fungizide Aktivität besitzen (JP-OS 40741/1975, entsprechend der DE-OS 23 33 354 [Le A 15 148]). Auch in dieser Veröffentlichung sind Aralkyl-Gruppen als mögliche Substituenten genannt; weder beschreiben diese Schriften jedoch solche Aralkyl-Gruppen, die der allgemeinen Formel gemäß der vorliegenden Erfindung entsprechen, noch legen sie sie nahe.

Nunmehr gefunden wurden neue 3-substituierte Azolylbutan-Derivate der nachstehenden Formel

$$Ar - \underset{R^1}{\overset{CH_3}{C}} - X - \underset{Az}{CH} - O - R^2 \qquad (I)$$

,

in der

Ar eine Phenyl-Gruppe bezeichnet, die durch Halogen substituiert sein kann,

$R^1$ eine Alkyl- oder eine Phenyl-Gruppe bezeichnet,

X $-\overset{OH}{CH}-$ oder $-\overset{O}{\overset{\|}{C}}-$ bezeichnet,

Az eine 1-Imidazolyl-Gruppe oder eine 1,2,4-Triazol-1-yl-Gruppe bezeichnet, und

$R^2$ eine Alkyl-Gruppe, eine Cycloalkyl-Gruppe oder eine Phenyl-Gruppe bezeichnet, die durch Halogen-Atome, Alkyl-Gruppen und gegebenenfalls halogen-substituierte Phenyl-Gruppen substituiert sein kann.

Weiterhin wurde gefunden, daß solche 3-substituierten Azolyl-butan-Derivate, die der Formel

$$Ar - \underset{R^1}{\overset{CH_3}{C}} - CO - \underset{Az}{CH} - O - R^2 \qquad (Ia)$$

entsprechen (Verbindungen der allgemeinen Formel (I), in der X -CO- ist), in der Ar, $R^1$, Az und $R^2$ die im Vorstehenden angegebene Bedeutung haben, durch Reaktion einer Verbindung der allgemeinen Formel

$$Ar - \underset{R^1}{\overset{CH_3}{C}} - CO - \underset{Hal}{CH} - O - R^2 \qquad (II)$$

,

in der Ar, $R^1$ und $R^2$ die im Vorstehenden angegebene Bedeutung haben und Hal ein Halogen-Atom, vorzugsweise Chlor und Brom, darstellt,
mit einer Verbindung der Formel

$$Az - H \qquad (III),$$

in der Az die im Vorstehenden angegebene Bedeutung hat, erhalten werden können.

Diejenigen 3-substituierten Azolyl-butan-Derivate, die der Formel

$$Ar - \underset{R^1}{\overset{CH_3}{C}} - \overset{OH}{CH} - \underset{Az}{CH} - O - R^2 \qquad (Ib)$$

entsprechen (Verbindungen der allgemeinen Formel (I), in der X -CH(OH)- ist), in der Ar, $R^1$, Az und $R^2$ die im Vorstehenden angegebene Bedeutung haben, werden durch Reduktion der Verbindung der oben angegebenen allgemeinen Formel (Ia) mit Reduktionsmitteln erhalten.

Die vorliegende Erfindung erstreckt sich auch auf Fungizide für Landwirtschaft und Gartenbau, die die neuen 3-substituierten Azolyl-butan-Derivate der allgemeinen Formel (I) als Wirkstoffe enthalten.

Die Verbindungen der oben angegebenen Formel (I) sind in der bisherigen Literatur nicht beschrieben und auch nicht in den oben zitierten Patent-Dokumenten speziell offenbart. Sie zeigen bessere fungizide Aktivität in Landwirtschaft und Gartenbau als herkömmliche analoge Verbindungen.

Wie oben festgestellt wurde sind die durch die Formel (I) dargestellten, biologisch aktiven Verbindungen gemäß der vorliegenden Erfindung in den oben zitierten Patent-Dokumenten nicht speziell offenbart, noch werden sie von den dort angegebenen allgemeinen Formeln mit erfaßt. Es wurde gefunden, daß diese Verbindungen gemäß der vorliegenden Erfindung unerwartete und in überraschender Weise hervorragende biologische Aktivität

zeigen, wie im Folgenden durch den Vergleich mit den biologischen Aktivitäten der Verbindungen gezeigt wird, die in den oben diskutierten Patent-Veröffentlichungen des Standes der Technik speziell offenbart und empfohlen wurden.

Die 3-substituierten Azolyl-butan-Derivate gemäß der vorliegenden Erfindung werden durch die allgemeine Formel (I) bezeichnet. Bevorzugt sind solche Verbindungen der Formel (I), in denen

Ar eine Phenyl-Gruppe bezeichnet, die durch Fluor, Chlor oder Brom substituiert sein kann,

$R^1$ eine Methyl- oder eine Phenyl-Gruppe bezeichnet,

X eine Gruppe $-\overset{OH}{\overset{|}{C}}H-$ oder $-\overset{O}{\overset{\|}{C}}-$ bezeichnet,

Az eine 1-Imidazolyl-Gruppe oder eine 1,2,4-Triazol-1-yl-Gruppe bezeichnet, und

$R^2$ eine Alkyl-Gruppe mit 1 bis 4 Kohlenstoff-Atomen, eine Cycloalkyl-Gruppe mit 5 oder 6 Kohlenstoff-Atomen oder eine Phenyl-Gruppe bezeichnet, die substituiert sein kann durch Fluor, Chlor oder Brom, durch Methyl-, Ethyl-, Propyl- oder Butyl-Gruppen oder durch eine Phenyl-Gruppe, die durch Chlor substituiert sein kann.

Einige spezielle Beispiele für Verbindungen der allgemeinen Formel (I) seien hier angegeben:

1-(4-Chlorophenoxy)-1-(1,2,4-triazol-1-yl)-3-methyl-3-phenyl-butan-2-on,

1-Phenoxy-1-(1,2,4-triazol-1-yl)-3-methyl-3-(3-chlorophenyl)-butan-2-on,

1-(4-Chlorophenoxy)-1-(1,2,4-triazol-1-yl)-3-methyl-3-(3-chlorophenyl)-butan-2-on,
1-(4-Chlorophenoxy)-1-(1,2,4-triazol-1-yl)-3-methyl-3-(4-chlorophenyl)-butan-2-on,
1-(4-Chlorophenoxy)-1-(1-imidazolyl)-3-methyl-3-(4-chlorophenyl)-butan-2-on,
1-(4-Phenylphenoxy)-1-(1,2,4-triazol-1-yl)-3-methyl-3-(4-chlorophenyl)-butan-2-on,
1-(4-Phenylphenoxy)-1-(1-imidazolyl)-3-methyl-3-(4-chlorophenyl)-butan-2-on,
1-Phenoxy-1-(1,2,4-triazol-1-yl)-3,3-diphenylbutan-2-on und
1-Phenoxy-1-(1-imidazolyl)-3,3-diphenylbutan-2-on
sowie die entsprechenden Butan-2-ol-Derivate.

Die Herstellung einer Verbindung der allgemeinen Formel (Ia) (die Formel (Ia) repräsentiert Keto-Verbindungen der allgemeinen Formel (I), in denen X eine -CO- Gruppe ist), kann durch die nachstehende Reaktionsgleichung erläutert werden; als Beispiel angegeben ist die Herstellung von 1-(4-Chlorophenoxy)-1-(1,2,4-triazol-1-yl)-3-methyl-3-phenyl-butan-2-on:

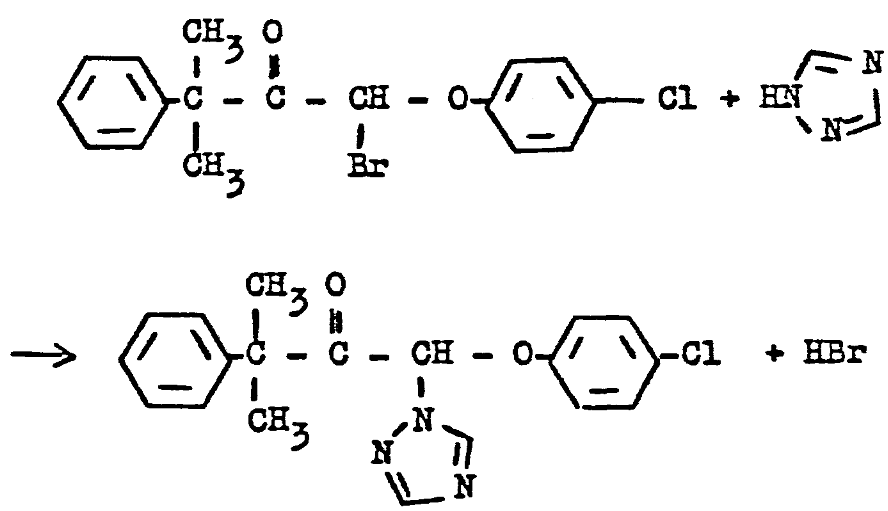

Die Herstellung einer Verbindung der allgemeinen Formel (Ib) (die Formel (Ib) repräsentiert solche Verbindungen der allgemeinen Formel (I), in denen X eine -CH(OH)-Gruppe ist), kann durch die nachstehende Reaktionsgleichung erläutert werden; als Beispiel angegeben ist die Herstellung von 1-(4-Chlorophenoxy)-1-(1,2,4-triazol-1-yl)-3-methyl-3-(chlorophenyl)-butan-2-ol:

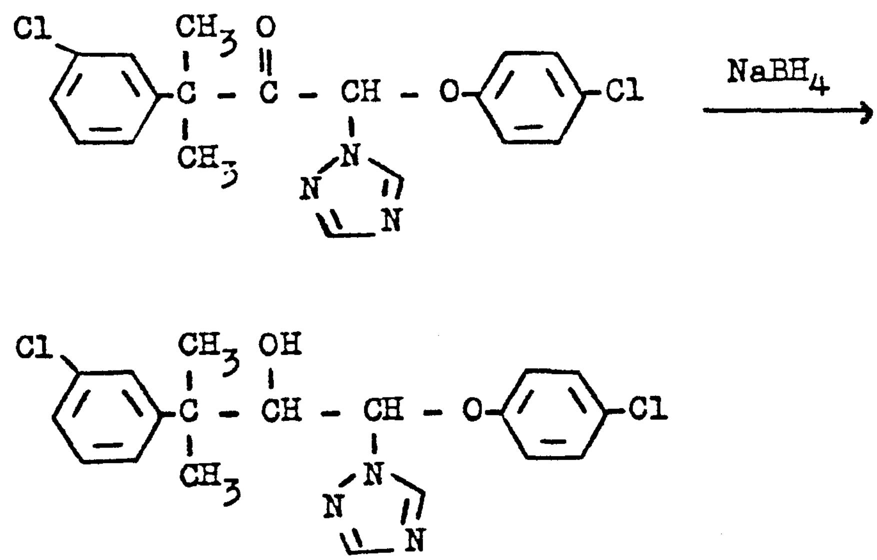


Die in dem Verfahren zur Herstellung der Verbindungen der allgemeinen Formel (I) gemäß der vorliegenden Erfindung eingesetzten Ausgangs-Verbindungen der allgemeinen Formel (II) sind neue Verbindungen, die in vor dem Einreichen der vorliegenden Anmeldung erschienenen Druckschriften nicht beschrieben sind. Diese neuen Ausgangs-Verbindungen der allgemeinen Formel (II) werden durch Reaktion einer Halogenoalkanon-Verbindung der Formel (IV) mit einem Alkanol der Formel (V) und nachfolgende Halogenierung der Ether-Verbindung (VI) mit Halogen (VII) erhalten:

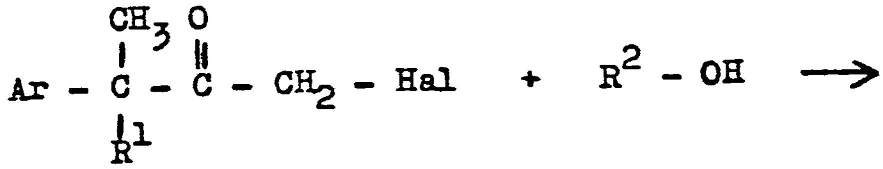

(IV) (V)

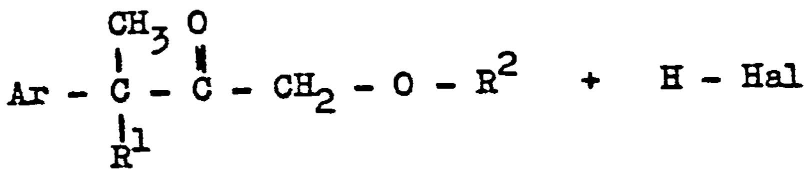

(VI)

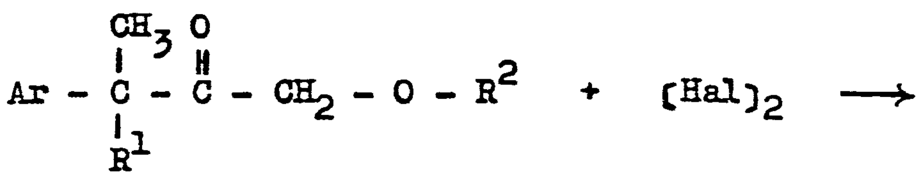

(VI) (VII)

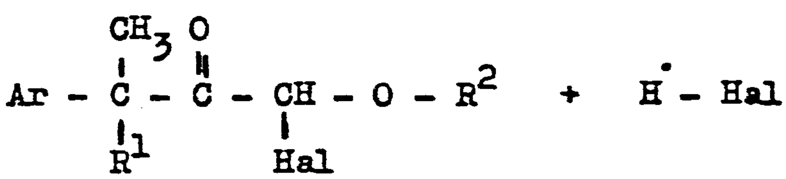

(II)

In den Formeln sind Ar, $R^1$, $R^2$, Az und Hal die gleichen, wie sie im Vorstehenden definiert wurden.

Spezielle Beispiele für die Ausgangs-Verbindungen der allgemeinen Formel (II) sind:

1-Bromo-1-phenoxy-3-methyl-3-phenylbutan-2-on,
1-Bromo-1-(2-chlorophenoxy)-3-methyl-3-phenylbutan-2-on,
1-Bromo-1-(3-chlorophenoxy)-3-methyl-3-phenylbutan-2-on,
1-Bromo-1-(4-chlorophenoxy)-3-methyl-3-phenylbutan-2-on,
1-Bromo-1-(4-methylphenoxy)-3-methyl-3-phenylbutan-2-on,
1-Bromo-1-(2,4-dichlorophenoxy)-3-methyl-3-phenylbutan-2-on,
1-Bromo-1-(2,4,6-trichlorophenoxy)-3-methyl-3-phenylbutan-2-on,
1-Bromo-1-phenoxy-3-(4-chlorophenyl)-3-methyl-butan-2-on,
1-Bromo-1-phenoxy-3-(2,4-dichlorophenyl)-3-methylbutan-2-on,
1-Bromo-1-propoxy-3-methyl-3-phenyl-butan-2-on,
1-Bromo-1-cyclohexyloxy-3-methyl-3-phenyl-butan-2-on,
1-Bromo-1-phenoxy-3-(2-chlorophenyl)-3-methyl-butan-2-on,
1-Bromo-1-phenoxy-3-(3-chlorophenyl)-3-methyl-butan-2-on,
1-Bromo-1-(4-chlorophenoxy)-3-(3-chlorophenyl)-3-methyl-butan-2-on,
1-Bromo-1-(4-chlorophenoxy)-3-(4-chlorophenyl)-3-methyl-butan-2-on,
1-Bromo-1-(4-phenylphenoxy)-3-(4-chlorophenyl)-3-methyl-butan-2-on und
1-Bromo-1-phenoxy-3,3-diphenyl-butan-2-on.

Die entsprechenden 1-Chloro-Verbindungen können auch als Beispiele genannt werden.

Spezielle Beispiele für die Verbindung der allgemeinen Formel (III), die gleichermaßen ein Ausgangsstoff ist, sind Imidazol und 1H-1,2,4-Triazol.

Spezielle Beispiele für die Verbindungen der allgemeinen Formeln (IV), (V), (VI) und (VII), die die zur Herstellung der Verbindungen der Ausgangsstoffe (II) eingesetzten Ausgangsmaterialien sind, sind im Folgenden angegeben:

Verbindungen der allgemeinen Formel (IV):

α,α-Dimethylbenzyl-bromomethylketon,
α,α-Dimethyl-4-chlorobenzyl-bromomethylketon,
α,α-Dimethyl-2,4-dichlorobenzyl-bromomethylketon,
α,α-Dimethyl-2-chlorobenzyl-bromomethylketon,
α,α-Dimethyl-3-chlorobenzyl-bromomethylketon und
1,1-Diphenylethylbromomethylketon.

Verbindungen der allgemeinen Formel (V):

Phenol,
2-Chlorophenol,
3-Chlorophenol,
4-Chlorophenol,
4-Methylphenol,
2,4-Dichlorophenol,
2,4,6-Trichlorophenol,
Propanol,
Cyclohexanol und
4-Phenylphenol.

Verbindungen der allgemeinen Formel (VI):

α,α-Dimethylbenzyl-phenoxymethylketon,
α,α-Dimethylbenzyl-2-chlorophenoxymethylketon,
α,α-Dimethylbenzyl-3-chlorophenoxymethylketon,
α,α-Dimethylbenzyl-4-chlorophenoxymethylketon,
α,α-Dimethylbenzyl-4-methylphenoxymethylketon,
α,α-Dimethylbenzyl-2,4-dichlorophenoxymethylketon,
α,α-Dimethylbenzyl-2,4,6-trichlorophenoxymethylketon,
α,α-Dimethyl-4-chlorobenzyl-phenoxymethylketon,
α,α-Dimethyl-2,4-dichlorobenzyl-phenoxymethylketon,
α,α-Dimethylbenzylcyclohexyloxymethylketon,
α,α-Dimethyl-2-chlorobenzyl-phenoxymethylketon,
α,α-Dimethyl-3-chlorobenzyl-phenoxymethylketon,
α,α-Dimethyl-3-chlorobenzyl-4-chlorophenoxymethylketon
α,α-Dimethyl-4-chlorobenzyl-4-chlorophenoxymethylketon
α,α-Dimethyl-4-chlorobenzyl-4-phenylphenoxymethylketon
und
1,1-Diphenylethylphenoxymethylketon.

Die vorstehend aufgeführten Verbindungen der allgemeinen Formel (VI) sind ebenfalls neue Verbindungen.

Verbindungen der Formel (VII):

Brom und Chlor.

An Stelle der oben genannten Halogene kann Sulfurylchlorid verwendet werden.

Als typisches Beispiel für die Herstellung einer Ausgangs-Verbindung der allgemeinen Formel (II) wird die Herstellung von 1-Bromo-1-(4-chlorophenoxy)-3-methyl-3-phenylbutan-2-on dargestellt:

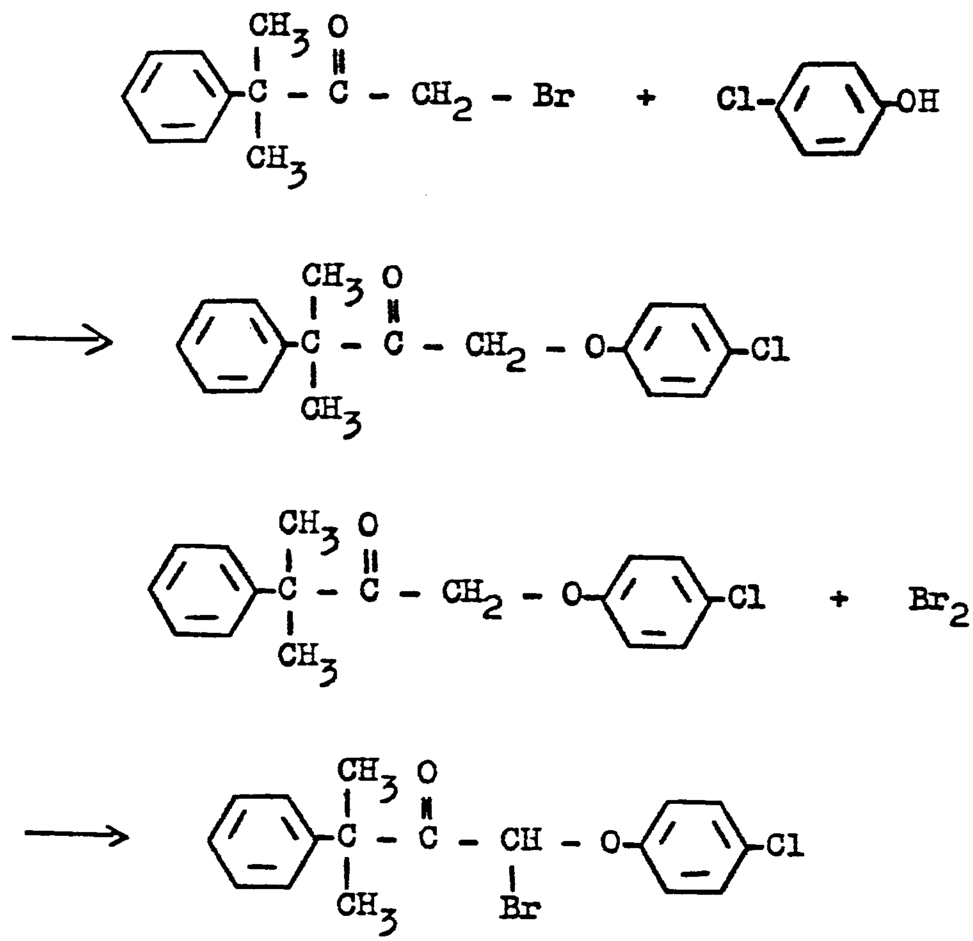

Weitere Einzelheiten in bezug auf die Herstellung der Ausgangs-Verbindungen sind im experimentellen Teil angegeben.

Das Verfahren zur Herstellung der Keto-Verbindungen der allgemeinen Formel (Ia) gemäß der vorliegenden Erfindung kann zweckmäßigerweise unter Verwendung eines Lösungsmittels oder Verdünnungsmittels durchgeführt werden. Zu diesem Zweck können sämtliche inerten Lösungsmittel und Verdünnungsmittel verwendet werden.

Zu Beispielen für solche Lösungsmittel oder Verdünnungsmittel zählen Wasser, aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Die obige Reaktion kann in Gegenwart eines säurebindenden Mittels durchgeführt werden. Beispiele für das säurebindende Mittel sind die Hydroxide, Carbonate und Hydrogencarbonate von Alkalimetallen sowie tertiäre Amine wie Triethylamin, Diethylanilin und Pyridin, die normalerweise verwendet werden.

Das Verfahren gemäß der vorliegenden Erfindung kann in einem weiten Temperaturbereich durchgeführt werden. Beispielsweise kann es bei einer Temperatur zwischen etwa -20°C und dem Siedepunkt der Mischung, vorzugsweise zwischen etwa 0°C und etwa 100°C, durchgeführt werden. Die Reaktion wird zweckmäßigerweise unter Atmosphärendruck durchgeführt, jedoch ist es auch möglich, unter erhöhtem oder vermindertem Druck zu arbeiten.

Das Verfahren zur Herstellung der durch die allgemeine Formel (Ib) bezeichneten Alkanol-Verbindungen gemäß der vorliegenden Erfindung kann mit beliebigen Reduktionsmitteln durchgeführt werden, die allgemein zur Anwendung kommen. Zu speziellen Beispielen zählen Hydride wie Natriumborhydrid und Lithiumaluminiumhydrid; neben diesen können Aluminiumisopropylat oder Wasserstoff in Gegenwart eines Katalysators als Reduktionsmittel verwendet werden. Wenn ein Hydrid wie Natriumborhydrid benutzt wird, wird die Reaktion vorzugsweise in Gegenwart eines polaren Lösungsmittels durchgeführt. Bevorzugt werden hierfür Alkohole wie Methanol verwendet.

Die Verbindungen der allgemeinen Formel (Ia) (wobei X in der Formel (I) -CO- ist) können auch mittels der alternativen Verfahren a) oder b) hergestellt werden, die schematisch hiernach dargestellt sind.

<u>Alternativ-Verfahren a):</u>

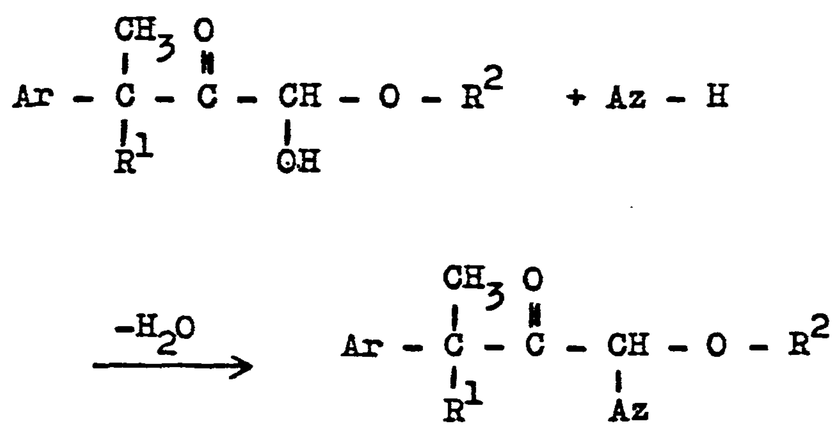


(Ia)

Alternativ-Verfahren b):

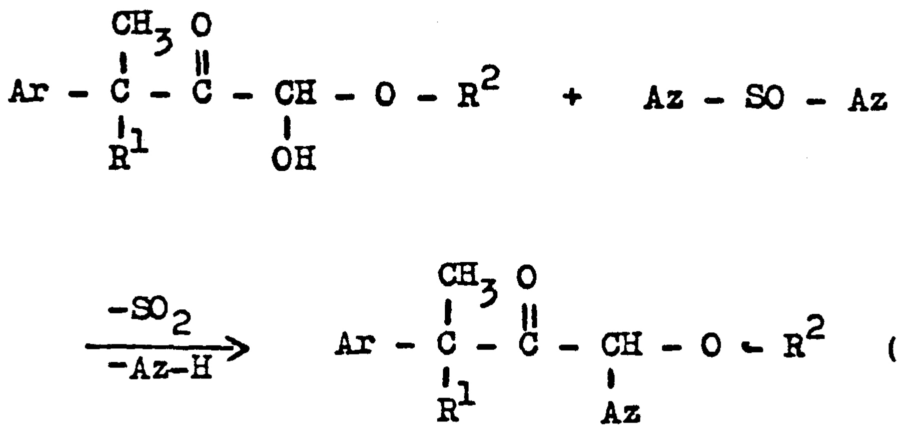


(Ia)

In dem Alternativ-Verfahren b) kann Az-CO-Az an Stelle von Az-SO-Az eingesetzt werden.

Die Verbindungen der allgemeinen Formel (II), die Zwischenprodukte sind, können durch nacheinander ablaufende Reaktionen ohne Abtrennung der Verbindungen der allgemeinen Formel (VI), die ebenfalls Zwischenprodukte sind, hergestellt werden. Weiterhin können die Verbindungen der allgemeinen Formel (I) gemäß der vorliegenden Erfindung durch Reaktionsfolgen aus den Ausgangs-Verbindungen der allgemeinen Formel (IV) hergestellt werden. Die Verbindungen der allgemeinen Formel (I) gemäß der vorliegenden Erfindung können aber auch durch Reaktionsfolgen aus den Ausgangs-Verbindungen der allgemeinen Formel (II) hergestellt werden, nachdem die Verbindungen der allgemeinen Formel (VI) abgetrennt wurden.

Die gleichen Lösungsmittel, die im Vorstehenden im Hinblick auf die Herstellung der Verbindungen der allgemeinen Formel (Ia) aufgeführt sind, können auch für die Herstellung der Zwischenpprodukte der allgemeinen Formel (II) verwendet werden. Das Herstellungsverfahren kann innerhalb eines breiten Temperaturbereichs durchgeführt werden.

Das charakteristische Merkmal der durch die allgemeine Formel (I) bezeichneten Verbindungen gemäß der vorliegenden Erfindung liegt in ihrer ausgezeichneten und einmaligen biologischen Aktivität, die ihrer charakteristischen chemischen Struktur zugeschrieben wird. Wie aus der allgemeinen Formel (I) hervorgeht, ist das charakteristische Merkmal der Verbindungen gemäß der vorliegenden Erfindung die Art der Substitution in einem 1-Azolyl-butan-Derivat an dem Kohlenstoff-Atom in der 3-Stellung der Butan-Kette. Es wurde gefunden, daß eine Korrelation zwischen der genannten chemischen Struktur der durch die allgemeine Formel (I) dargestellten Verbindungen und ihrer biologischen Aktivität besteht, und infolgedessen zeigen die Verbindungen unerwartete und überraschende Effekte.

Beim Einsatz als Fungizid in Landwirtschaft und Gartenbau zeigen die Verbindungen gemäß der vorliegenden Erfindung eine besonders gute biologische Aktivität. Sie sind besonders gekennzeichnet durch eine sehr bemerkenswerte, genaue Bekämpfungswirkung gegen pulvrigen Mehltau, der weitverbreitet auf allgemeinen Pflanzen auftritt, während sie bei den Pflanzen selbst keine Phytotoxizität verursachen.

Das fungizide Spektrum der Verbindungen gemäß der vorliegenden Erfindung zeigt, daß sie wirksam gegen verschiedene Pflanzenerkrankungen eingesetzt werden können, die durch Archimycetes, Phycomycetes, Ascomycetes, Basidiomycetes und Fungi imperfecti sowie Bakterien verursacht werden. Insbesondere zeigen die Verbindungen gemäß der vorliegenden Erfindung eine sehr ausgeprägte fungizide Wirkung gegen verschiedene Arten von pulvrigem Mehltau, die bei verschiedenen Pflanzen auftreten (beispielsweise Sphaerotheca fuliginea), und gegen die Brusone-Krankheit von Reis (Piricularia oryzae). Sie zeigen auch eine hervorragende Bekämpfungswirkung gegen Grauschimmel (Botrytis cinerea) und Rost (Puccinia recondita) auf verschiedenen Nutzpflanzen sowie Schalenmehltau (Pellicularia sasakii) und bakteriellen Blattbrand (Xanthomonas oryzae) bei Reis.

Als Fungizide für Landwirtschaft und Gartenbau können die durch die Formel (I) bezeichneten Verbindungen gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht.

Beispiele für die landwirtschaftlich unbedenklichen Hilfsstoffe, auf die hier Bezug genommen wird, umfassen Verdünnungsmittel (Lösungsmittel, Streckmittel, Träger), oberflächenaktive Mittel (Lösungsvermittler,

Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /¯z.B. n-Hexan, Petrolether, Erdöl-Fraktionen (z.B. Paraffinwachse, Kerosin, Leichtöle, Mittelöle und Schweröle), Benzol, Toluol und Xylol_7, halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methylalkohol, Ethylalkohol, Propylalkohol und Ethylenglycol), Ether (z.B. Diethylether, Ethylenoxid und Dioxan), Alkoholether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z.B. Natriumlaurylsulfonat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchloride) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol und niedere Ether), die Verbrennung steuernde Mittel für Räucherpräparate (z.B. Nitrite, Zink-Pulver und Dicyandiamid), Sauerstoff abgebende Mittel (z.B. Chlorate), wirkungsverlängernde Mittel, Dispersions-Stabilisatoren /‾z.B. Casein, Tragant, Carboxymethylcellulose und Polyvinylalkohol_7 und synergistische Mittel.

Die Verbindungen gemäß der vorliegenden Erfindung können mittels allgemein bei der Herstellung von Agrochemikalien angewandter Methoden zu verschiedenartigen

Präparaten formuliert werden. Zu Beispielen für solche Anwendungsformen zählen emulgierbare Konzentrate, Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Das Fungizid für Landwirtschaft und Gartenbau gemäß der vorliegenden Erfindung kann etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats, dem Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen /¯z.B. Organophosphat-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Streuen, Bedampfen, Gießen etc.), Oberflächenbehandlung (Beschichten, Bändern, Bestäuben, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein fungizides Mittel für die Landwirtschaft verfügbar gemacht werden, das eine Verbindung der allgemeinen Formel (I) als Wirkstoff und ein Verdünnungsmittel (ein Lösungsmittel und/oder ein Streckmittel und/oder einen Träger) und/ oder ein oberflächenaktives Mittel und, sofern weiter erforderlich, einen Stabilisator, ein Haftmittel, ein synergistisches Mittel etc. enthält.

Die vorliegende Erfindung stellt ebenfalls ein Verfahren zur Bekämpfung von Pflanzenkrankheiten zur Verfügung, bei dem auf die Krankheitserreger und/oder deren

Lebensraum und den Ort des Auftretens der Erkrankungen die Verbindung der Formel (I) allein oder im Gemisch mit einem Verdünnungsmittel (einem Lösungsmittel und/ oder einem Streckmittel und/oder einem Träger) und/oder einem oberflächenaktiven Mittel und, sofern weiter erforderlich, einem Stabilisator, einem Haftmittel, einem synergistischen Mittel etc. aufgebracht wird.

Beispiele für das Herstellungsverfahren:

Beispiel 1
(Verbindung Nr. 1)

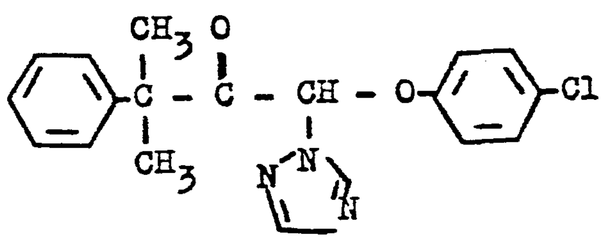

α,α-Dimethylbenzyl-4-chlorophenoxymethylketon (5,76 g) wurde in Kohlenstofftetrachlorid (30 ml) gelöst, und Brom (3,2 g) wurde tropfenweise bei einer Temperatur zwischen 0°C und 10°C zu der Lösung hinzugefügt. Nach der Zugabe wurde die Mischung 1 h gerührt, und das Kohlenstofftetrachlorid wurde bei 20°C unter vermindertem Druck abgedampft. Der Rückstand wurde in Acetontril (30 ml) gelöst, und Triazol (5,5 g) wurde zugegeben. Die Mischung wurde 3 h unter Rückfluß erhitzt. Nach der Reaktion wurde das Acetonitril unter vermindertem Druck abgedampft. Der Rückstand wurde in Toluol (50 ml) gelöst. Die Toluol-Schicht wurde mit Wasser gut gewaschen und über Natriumsulfat getrocknet. Das Toluol wurde unter vermindertem Druck abgedampft.

Umkristallisieren des Rückstandes aus Ethanol lieferte das gewünschte 1-(4-Chlorophenoxy)-1-(1,2,4-triazol-1-yl)-3-methyl-3-phenyl-butan-2-on (5,1 g) mit dem Schmp. 168-170°C.

Beispiel 2

(Verbindung Nr. 2)

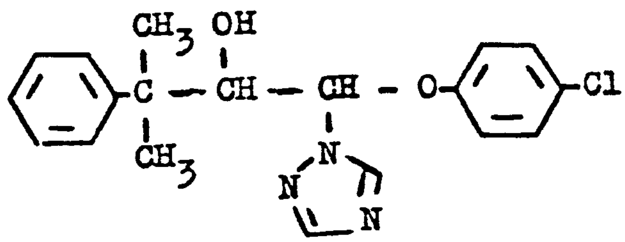

1-(4-Chlorophenoxy)-1-(1,2,4-triazol-1-yl)-3-methyl-3-phenyl-butan-2-on (1 g) wurde in Methanol (10 ml) gelöst, und Natriumborhydrid (0,3 g) wurde bei 0°C in kleinen Portionen hinzugefügt. Nach der Zugabe wurde die Mischung 3 h gerührt. Wasser (50 ml) wurde der Reaktionsmischung zugesetzt, und sie wurde danach mit Diethylether extrahiert. Die Ether-Schicht wurde gut mit Wasser gewaschen und über Natriumsulfat getrocknet. Der Ether wurde abgedampft, und der Rückstand wurde aus einer Mischung aus Hexan und Ether (3:1) umkristallisiert, wonach das gewünschte 1-(4-Chlorophenoxy)-1-(1,2,4-triazol-1-yl)-3-methyl-3-phenyl-butan-2-ol (570 mg) mit einem Schmp. 130-133°C erhalten wurde.

Nach der gleichen Arbeitsweise wie in Beispiel 1 wurden die in der folgenden Tabelle 1 aufgeführten Verbindungen synthetisiert.

Tabelle 1

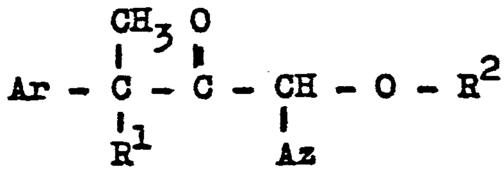

| Verbindung Nr. | Ar | $R^1$ | Az | $R^2$ | Physikal. Konstante |
|---|---|---|---|---|---|
| 3 | 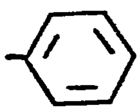 | $-CH_3$ | 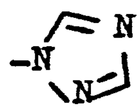 | 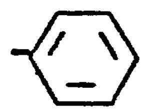 | Schmp.150-152°C |
| 4 | 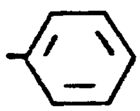 | $-CH_3$ | 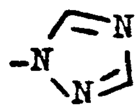 | 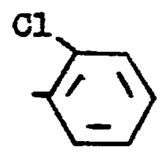 | Schmp.123-124°C |
| 5 | 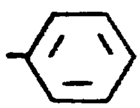 | $-CH_3$ | 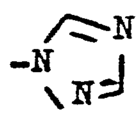 | 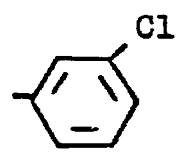 | Schmp.142-143°C |
| 6 | 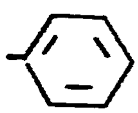 | $-CH_3$ | 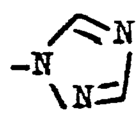 | 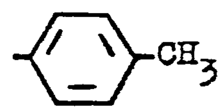 | Schmp.160-162°C |
| 7 | 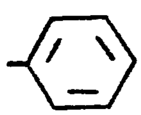 | $-CH_3$ | 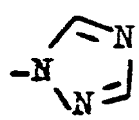 | 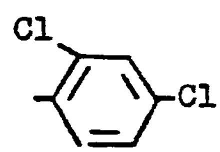 | Schmp.122-124°C |
| 8 | 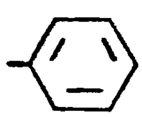 | $-CH_3$ | 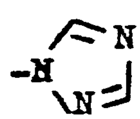 | 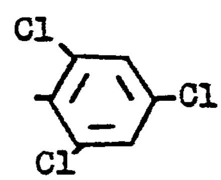 | Schmp.134-136°C |
| 9 | 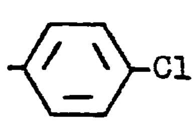 | $-CH_3$ | 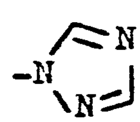 | 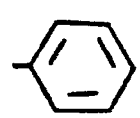 | Schmp.117-119°C |

Tabelle 1 - Fortsetzung

| Verbindung Nr. | Ar | $R^1$ | Az | $R^2$ | Physikal. Konstante |
|---|---|---|---|---|---|
| 10 | 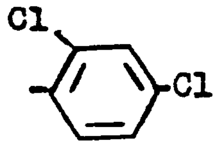 | $-CH_3$ | 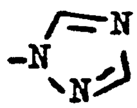 | 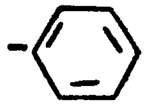 | Schmp.125-127°C |
| 11 | 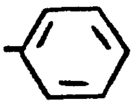 | $-CH_3$ | 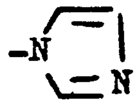 | 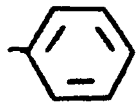 | $n_D^{20}$ 1,5339 |
| 12 | 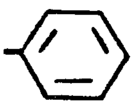 | $-CH_3$ | 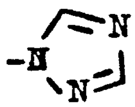 | $-C_3H_7$-n | glasig |
| 13 | 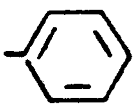 | $-CH_3$ | 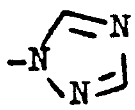 | 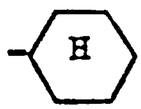 | $n_D^{20}$ 1,5289 |
| 14 | 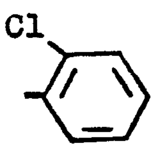 | $-CH_3$ | 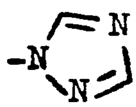 | 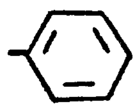 | glasig |
| 15 | 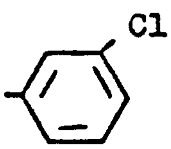 | $-CH_3$ | 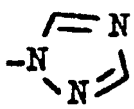 | 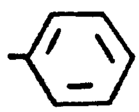 | glasig |
| 16 | 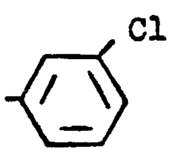 | $-CH_3$ | 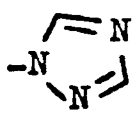 | 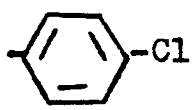 | glasig |
| 17 | 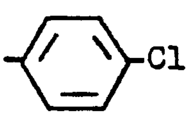 | $-CH_3$ | 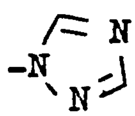 | 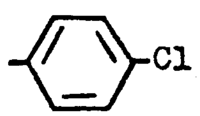 | Schmp.115-117°C |
| 18 | 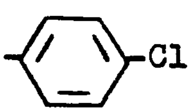 | $-CH_3$ | 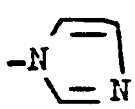 | 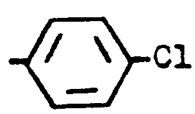 | Schmp.121-124°C |

Tabelle 1 - Fortsetzung

| Verbindung Nr. | Ar | $R^1$ | Az | $R^2$ | Physikal. Konstante |
|---|---|---|---|---|---|
| 19 | 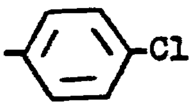 | $-CH_3$ | 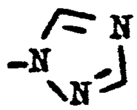 | 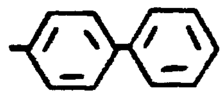 | $n_D^{20}$ 1,5800 |
| 20 | 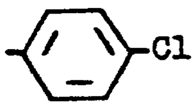 | $-CH_3$ | 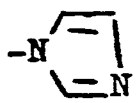 | 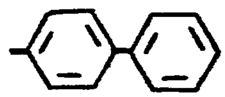 | $n_D^{20}$ 1,5350 |
| 21 | 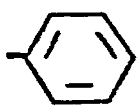 | 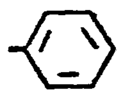 | 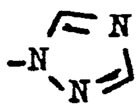 | 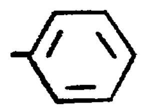 | Schmp.122-125°C |
| 22 | 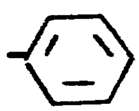 | 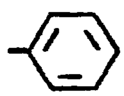 | 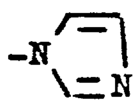 | 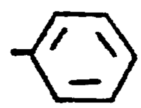 | glasig |

Die nachstehende Tabelle 2 zeigt die in gleicher Weise wie in Beispiel 2 synthetisierten Verbindungen gemäß der vorliegenden Erfindung.

Tabelle 2

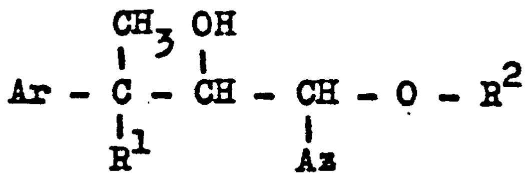

| Verbindung Nr. | Ar | $R^1$ | Az | $R^2$ | Physikal. Konstante |
|---|---|---|---|---|---|
| 23 | 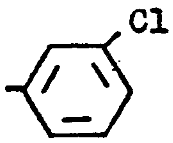 | $-CH_3$ | 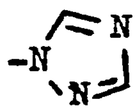 | 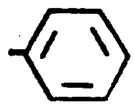 | Schmp.117-120°C |
| 24 | 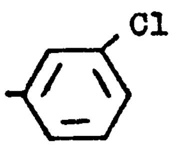 | $-CH_3$ | 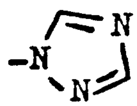 | 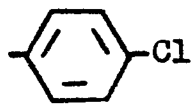 | Schmp.110-112°C |
| 25 | 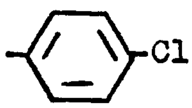 | $-CH_3$ | 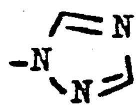 | 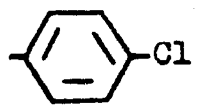 | Schmp.144-146°C |
| 26 | 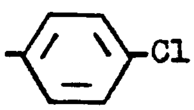 | $-CH_3$ | 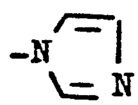 | 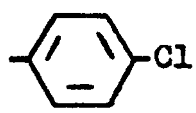 | Schmp.115-118°C |
| 27 | 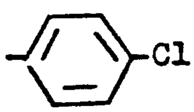 | $-CH_3$ | 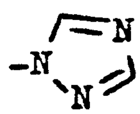 | 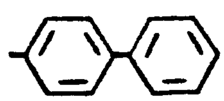 | Schmp.155-158°C |
| 28 | 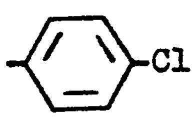 | $-CH_3$ | 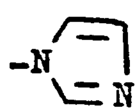 | 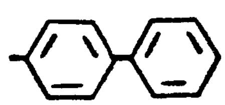 | Schmp.117-120°C |

Tabelle 2 - Fortsetzung

| Verbindung Nr. | Ar | $R^1$ | Az | $R^2$ | Physikal. Konstante |
|---|---|---|---|---|---|
| 29 | 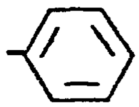 | 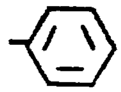 | 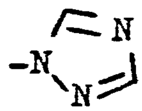 | 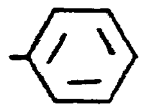 | Schm.117-119°C |
| 30 | 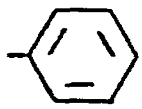 | 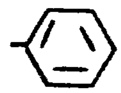 | 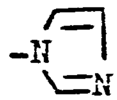 | 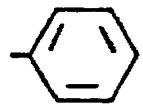 | glasig |

Synthese der Ausgangsstoffe

Verbindung VI-1:

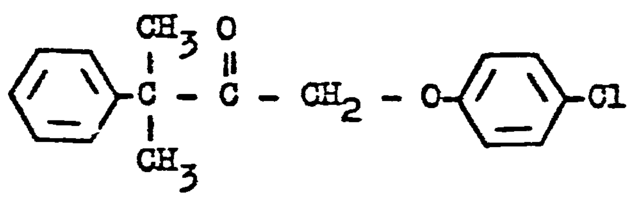

α,α-Dimethylbenzylbromomethylketon (8,16 g) und 4-Chlorophenol (3,3 g) wurden zusammen mit wasserfreiem Kaliumcarbonat in Acetonitril (50 ml) 16 h unter Rückfluß erhitzt. Die anorganischen Stoffe wurden durch Filtration entfernt, und Acetonitril wurde unter vermindertem Druck abgedampft. Der Rückstand wurde in Toluol (50 ml) gelöst und mit einer 1-proz. wäßrigen Natriumhydroxid-Lösung und mit Wasser in dieser Reihenfolge gewaschen. Die Toluol-Schicht wurde über wasserfreiem Natriumsulfat getrocknet, und das Toluol wurde unter vermindertem

Druck abgedampft. Das Waschen des Rückstandes mit Diethylether ergab α,α-Dimethylbenzyl-4-chlorophenoxymethylketon (6,2 g) mit einem Schmp. 79-80°C.

Nach dem gleichen Verfahren wie oben beschrieben synthetisierte Ausgangs-Verbindungen sind in der nachstehenden Tabelle 3 aufgeführt.

Tabelle 3

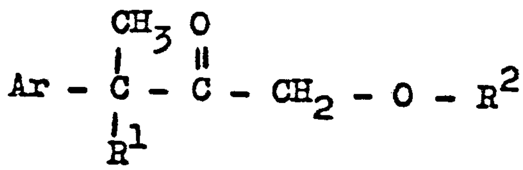

| Verbindung Nr. | Ar | $R^1$ | $R^2$ | Physikal. Konstante |
|---|---|---|---|---|
| VI-2 | 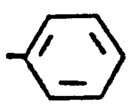 | $-CH_3$ | 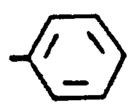 | Schmp.90-92°C |
| VI-3 | 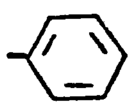 | $-CH_3$ | 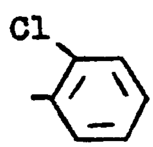 | Sdp.230-235°C /0,67 mbar (0,5 mmHg) |
| VI-4 | 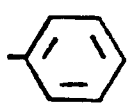 | $-CH_3$ | 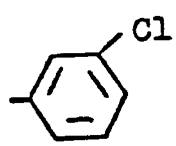 | Sdp.235-240°C /0,67 mbar (0,5 mmHg) |
| VI-5 | 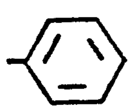 | $-CH_3$ | 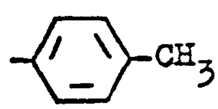 | Sdp.220-225°C /0,67 mbar (0,5 mmHg) |
| VI-6 | 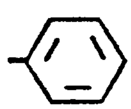 | $-CH_3$ | 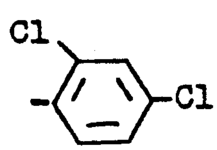 | Sdp.255-260°C 0,67 mbar (0,5 mmHg) |

Tabelle 3 - Fortsetzung

| Verbindung Nr. | Ar | $R^1$ | $R^2$ | Physikal. Konstante |
|---|---|---|---|---|
| VI-7 | 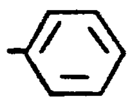 | $-CH_3$ | 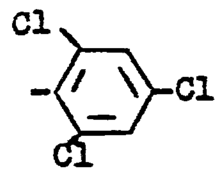 | Sdp.223-225°C /0,13 mbar (0,1 mmHg) |
| VI-8 | 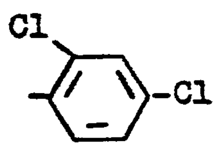 | $-CH_3$ | 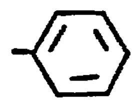 | Sdp.220-222°C /0,67 mbar (0,5 mmHg) |
| VI-9 | 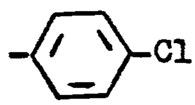 | $-CH_3$ | 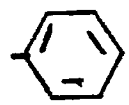 | Sdp.190-200°C /0,67 mbar (0,5 mmHg) |
| VI-10 | 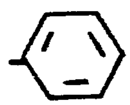 | $-CH_3$ | 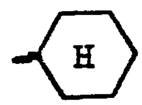 | Sdp. 95-100°C /0,13 mbar (0,1 mmHg) |

Formulierungsbeispiele:

Benetzbares Pulver:

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf einen Krankheitserreger und/oder seinen Lebensraum und den Ort, an dem eine Pflanzenkrankheit auftritt, aufgesprüht.

Emulgierbares Konzentrat:

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf einen Krankheitserreger und/oder seinen Lebensraum und den Ort, an dem eine Pflanzenkrankheit auftritt, aufgesprüht.

Stäubemittel:

2 Teile der Verbindung Nr. 3 der Erfindung und 98 Teile Tonpulver werden pulverisiert und gemischt, wodurch ein Stäubemittel hergestellt wird. Dieses wird über einem Krankheitserreger und/oder seinen Lebensraum und den Ort, an dem eine Pflanzenkrankheit auftritt, ausgestreut.

Stäubemittel:

Die Verbindung Nr. 4 der Erfindung (1,5 Teile), 0,5 Teile Isopropylhydrogenphosphat und 98 Teile Tonpulver werden pulverisiert und gemischt, wodurch ein Stäubemittel hergestellt wird. Dieses wird über einem Krankheitserreger und/oder seinem Lebensraum und dem Ort, an dem eine Pflanzenkrankheit auftritt, ausgestreut.

Granulat:

Wasser (25 Teile) wird zu einer Mischung aus 10 Teilen der Verbindung Nr. 3 der Erfindung, 30 Teilen Bentonit

(Montmorillonit), 58 Teilen Talkum und 2 Teilen eines Ligninsulfonat-Salzes zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet, und das Granulat wird bei 40°C bis 50°C getrocknet. Das Granulat wird über einem Krankheitserreger und/oder seinem Lebensraum und dem Ort, an dem eine Pflanzenkrankheit auftritt, ausgestreut.

Verwendungsbeispiele:

Beispiel A:

Test gegen pulvrigen Mehltau auf Gurken (Sphaerotheca fuliginea):

Ein wie in Beispiel 5 hergestelltes emulgierbares Konzentrat jeder der Test-Verbindungen wurde mittels einer Spritzpistole auf Gurken (Varietät: Tokiwajibae) aufgesprüht, die sich im 2-Blatt-Stadium befanden und in unglasierten Töpfen (9 cm) angezogen worden waren. Einen Tag nach dem Sprühen wurde eine Suspension von Sporen des betreffenden Krankheitserregers, (Sphaerotheca fuliginea) durch Sprühen auf die Pflanzen aufgebracht. Die Töpfe wurden dann in einem Raum mit konstanter Temperatur von 23°C stehen gelassen. Zehn Tage später wurde der Grad der Infizierung mit der Erkrankung aufgrund des Prozentsatzes der Verletzungsfläche bestimmt, und der Bekämpfungswert wurde berechnet.

$$\text{Bekämpfungs-Wert (\%)} = 100 - \frac{\text{Grad der Infektion einer behandelten Fläche}}{\text{Grad der Infektion einer unbehandelten Fläche}} \times 100$$

Die Ergebnisse sind in Tabelle A aufgeführt.

Nit 174

Tabelle A

Test gegen pulvrigen Mehltau auf Gurken (Sphaerotheca fuliginea)

| Verbindung Nr. | Wirkstoff-Konzentration ppm | Bekämpfungs-Wert (%) |
|---|---|---|
| 2 | 5 | 100 |
| 10 | 5 | 100 |
| 14 | 5 | 100 |
| 15 | 5 | 100 |
| 16 | 5 | 100 |
| 17 | 5 | 100 |
| 18 | 5 | 100 |
| 23 | 5 | 100 |
| 24 | 5 | 100 |
| 25 | 5 | 100 |
| 27 | 5 | 100 |
| 29 | 5 | 100 |
| Kontrolle: | | |
| A-1 | 5 | 50 |
| Chinomethionat | 62,5 | 95 |

Anmerkungen: Kontroll-Verbindung A-1:

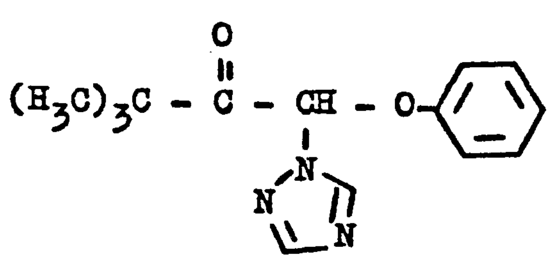

(eine in der JP-OS Nr. 80738/1973 bzw. der DE-OS 22 01 063 offenbarte Verbindung).

Die Kontroll-Verbindung "Chinomethionat" (Handelsprodukt) ist 6-Methyl-chinoxalin-2,3-dithiocarbonat (25 %, benetzbares Pulver).

Beispiel B:

Test der Nachwirkung gegen pulvrigen Mehltau auf Gurken (Sphaerotheca fuliginea):

Jede der aktiven Verbindungen wurde mittels der gleichen Verfahrensweise wie in Beispiel A gesprüht. Eine Woche nach dem Sprühen wurde mit der gleichen Sporensuspension des Krankheitserregers wie in Beispiel A inokuliert, und die Töpfe wurden in einem Raum mit konstanter Temperatur bei 23°C stehen gelassen. Zehn Tage später wurden die Bekämpfungswerte in gleicher Weise wie in Beispiel A berechnet. Die Ergebnisse sind in Tabelle B aufgeführt.

Tabelle B

Test der Nachwirkung gegen pulvrigen Mehltau auf Gurken (Sphaerotheca fuliginea)

| Verbindung Nr. | Wirkstoff-Konzentration ppm | Bekämpfungs-Wert (%) |
|---|---|---|
| 2 | 10 | 100 |
| 10 | 10 | 100 |
| 16 | 10 | 100 |
| 17 | 10 | 100 |
| 18 | 10 | 100 |
| 24 | 10 | 100 |
| 25 | 10 | 100 |
| Kontrolle: | | |
| A-1 | 10 | 35 |

Anmerkung:
Die Kontroll-Verbindung A-1 ist die gleiche wie in Tabelle A angegeben.

Beispiel C:
Test gegen die Brusone-Krankheit von Reis (Piricularia oryzae):

| | |
|---|---|
| Wirkstoff: | 50 Gew.-Teile; |
| Träger: | 45 Gew.-Teile einer Mischung (1 : 5) aus Diatomeenerde und Kaolin; |
| Emulgator: | 5 Gew.-Teile Polyoxyethylenalkylphenylether. |

Die aktive Verbindung, der Träger und der Emulgator wurden in den angegebenen Mengen pulverisiert und miteinander vermischt, wodurch ein benetzbares Pulver gebildet wurde. Eine vorher festgelegte Menge des benetzbaren Pulvers wurde mit Wasser verdünnt, wodurch ein Test-Präparat hergestellt wurde.

Überflutete Reispflanzen (Varietät: Asahi) wurden in unglasierten Töpfen mit einem Durchmesser von 12 cm kultiviert, und im 3- bis 4-Blatt-Stadium wurde eine wie oben auf eine vorher festgelegte Konzentration verdünnte Lösung der Test-Verbindung in einer Menge von 50 ml auf jeweils drei Töpfe aufgesprüht. Am nächsten Tag wurde eine Suspension von künstlich kultivierten Sporen des Brusone-Pilzes (Piricularia oryzae) durch (zweimaliges) Sprühen inokuliert, und die Töpfe wurden in einem feuchten Raum bei 25°C und einer relativen Luftfeuchtigkeit von 100 % gehalten, um die Infizierung hervorzurufen. Sieben Tage nach der Beimpfung wurde der

Grad der Erkrankung pro Topf aufgrund des folgenden Bewertungsmaßstabs beurteilt, und der Bekämpfungs-Wert wurde ebenfalls berechnet. Außerdem wurden Pflanzen auf Phytotoxizität untersucht.

| Grad der Erkrankung | Prozentuale Fläche der Verletzungen (%) |
|---|---|
| 0 | 0 |
| 0,5 | nicht mehr als 2 |
| 1 | 3 bis 5 |
| 2 | 6 bis 10 |
| 3 | 11 bis 20 |
| 4 | 21 bis 40 |
| 5 | wenigstens 41 |

$$\text{Bekämpfungs-Wert (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Im vorliegenden Test bestand eine Fläche aus drei Töpfen, und die für jede Fläche erhaltenen Ergebnisse sind in Tabelle C aufgeführt.

Tabelle C

Test gegen die Brusone-Krankheit von Reis (Piricularia oryzae):

| Verbindung Nr. | Wirkstoff-Konzentration ppm | Bekämpfungs-Wert (%) |
|---|---|---|
| 1 | 250 | 100 |
| 7 | 250 | 100 |
| 8 | 250 | 100 |
| 28 | 250 | 100 |
| Kontrolle: | | |
| A-1 | 250 | 20 |

Anmerkung:
Die Kontroll-Verbindung A-1 ist die gleiche wie in der Fußnote zu Tabelle A angegeben.

Beispiel D:
Topf-Test gegen Reis-Schalenmehltau (Pellicularia sasakii):

Wirkstoff: 50 Gew.-Teile;
Träger: 45 Gew.-Teile einer Mischung (1 : 5) aus Diatomeenerde und Kaolin;
Emulgator: 5 Gew.-Teile Polyoxyethylenalkylphenylether.

Die aktive Verbindung, der Träger und der Emulgator wurden in den angegebenen Mengen pulverisiert und miteinander vermischt, wodurch ein benetzbares Pulver gebildet wurde. Eine vorher festgelegte Menge des benetzbaren Pulvers wurde mit Wasser verdünnt, wodurch ein Test-Präparat hergestellt wurde.

Im Wasser wachsender Reis (Varietät: Kinmaze) wurde in Wagner-Töpfen im bewässerten Zustand kultiviert. Im frühen Stadium der Ähren-Bildung wurde die wie im Vorstehenden angegeben hergestellte Verdünnung des Test-Präparates in vorherbestimmter Konzentration in einer Menge von 100 ml auf jeweils 3 Töpfe aufgesprüht.

Am nächsten Tage wurden die Wurzeln der Reispflanzen mit (Pellicularia sasakii beimpft, der 10 Tage in einem Gerste-Medium bis zur Bildung von Sklerotia kultiviert worden war. Zwecks Infizierung wurden die Töpfe 10 Tage in einer feuchten Kammer gehalten, in der eine Temperatur von 28°C bis 30°C und eine relative Luftfeuchtigkeit von 95 % oder höher aufrechterhalten wurden. Danach wurde der Grad der Infektion untersucht. Die Prüfung erfolgte durch Messung des Grades der Ausdehnung der Schädigung von den beimpften Teilen der Wurzeln her, und der Grad der Schädigung wurde mit Hilfe des nachstehenden Standards ausgedrückt:

$$\text{Grad der Schädigung} = \frac{3n_3 + 2n_2 + n_1 + n_0}{3\,N} \times 100$$

Hierin bedeuten

N die Gesamtzahl der untersuchten Stengel,

$n_0$ die Gesamtzahl der durch die Erkrankung nicht infizierten Stengel,

$n_1$ die Zahl der Stengel, die (von unten her) bis hinauf zu einer Schale des ersten Blattes befallen waren,

$n_2$ die Zahl der Stengel, die (von unten her) bis hinauf zu einer Schale des zweiten Blattes befallen waren, und

$n_3$ die Zahl der Stengel, die (von unten her) bis hinauf zu einer Schale des dritten Blattes oder darüber hinaus befallen waren.

Die Ergebnisse sind in der Tabelle D dargestellt.

Tabelle D

Test gegen Reis-Schalenmehltau (Pellicularia sasakii):

| Verbindung Nr. | Wirkstoff-Konzentration (ppm) | Grad der Schädigung d. Hypochnus sasakii % |
|---|---|---|
| 10 | 125 | 0 |
| | 250 | 0 |
| 14 | 125 | 0 |
| | 250 | 0 |
| Kontrolle A-1 | 250 | 90 |

Anmerkung:
Die Kontroll-Verbindung A-1 ist die gleiche wie in der Fußnote zu Tabelle A angegeben.

<u>Patentansprüche</u>

1. 3-Substituierte Azolyl-butan-Derivate der allgemeinen Formel

$$Ar - \underset{R^1}{\overset{CH_3}{C}} - X - \underset{Az}{CH} - O - R^2 \qquad (I)$$

,

in der

Ar eine Phenyl-Gruppe bezeichnet, die durch Halogen substituiert sein kann,

$R^1$ eine Alkyl- oder eine Phenyl-Gruppe bezeichnet,

X eine Gruppe $-\overset{OH}{CH}-$ oder $-\overset{O}{\overset{\|}{C}}-$ bezeichnet,

Az eine 1-Imidazolyl-Gruppe oder eine 1,2,4-Triazol-1-yl-Gruppe bezeichnet, und

$R^2$ eine Alkyl-Gruppe, eine Cycloalkyl-Gruppe oder eine Phenyl-Gruppe bezeichnet, die durch Halogen-Atome, niedere Alkyl-Gruppen und gegebenenfalls halogen-substituierte Phenyl-Gruppen substituiert sein kann.

2. Verbindungen der Formel (I) nach Anspruch 1, dadurch gekennzeichnet, daß
   Ar eine Phenyl-Gruppe bezeichnet, die durch Fluor, Chlor oder Brom substituiert sein kann,
   $R^1$ eine Methyl- oder eine Phenyl-Gruppe bezeichnet,
   X eine -CH(OH)- oder -CO- Gruppe bezeichnet,
   Az eine 1-Imidazolyl-Gruppe oder eine 1,2,4-Triazol-1-yl-Gruppe bezeichnet, und
   $R^2$ eine Alkyl-Gruppe mit 1 bis 4 Kohlenstoff-Atomen, eine Cycloalkyl-Gruppe mit 5 oder 6 Kohlenstoff-Atomen oder eine Phenyl-Gruppe bezeichnet, die substituiert sein kann durch Fluor, Chlor oder Brom, durch Methyl-, Ethyl-, Propyl- oder Butyl-Gruppen oder durch eine Phenyl-Gruppe, die durch Chlor substituiert sein kann.

3. Verfahren zur Herstellung von 3-substituierten Azolylbutan-Derivaten der allgemeinen Formel

$$Ar - \overset{CH_3}{\underset{R^1}{C}} - X - \underset{Az}{CH} - O - R^2 \qquad (I)$$

,

in der
   Ar eine Phenyl-Gruppe bezeichnet, die durch Halogen substituiert sein kann,
   $R^1$ eine Alkyl- oder eine Phenyl-Gruppe bezeichnet,
   X eine -CH(OH)- oder -CO- Gruppe bezeichnet,
   Az eine 1-Imidazolyl-Gruppe oder eine 1,2,4-Triazol-1-yl-Gruppe bezeichnet, und
   $R^2$ eine Alkyl-Gruppe, eine Cycloalkyl-Gruppe oder

eine Phenyl-Gruppe bezeichnet, die durch Halogen-Atome, niedere Alkyl-Gruppen und gegebenenfalls halogen-substituierte Phenyl-Gruppen substituiert sein kann,

dadurch gekennzeichnet, daß eine Verbindung der allgemeinen Formel

$$\begin{array}{ccccccccc} & & CH_3 & & & & & & \\ & & \vert & & & & & & \\ Ar & - & C & - & CO & - & CH & - & O - R^2 \\ & & \vert & & & & \vert & & \\ & & R^1 & & & & Hal & & \end{array} \qquad (II)$$

,

in der

Ar, $R^1$ und $R^2$ die im Vorstehenden angegebene Bedeutung haben und

Hal ein Halogen-Atom darstellt,

mit einer Verbindung der Formel

$$Az - H \qquad (III),$$

in der Az die im Vorstehenden angegebene Bedeutung hat, umgesetzt wird und, falls erwünscht, die erhaltene Verbindung der allgemeinen Formel

$$\begin{array}{ccccccccc} & & CH_3 & & & & & & \\ & & \vert & & & & & & \\ Ar & - & C & - & CO & - & CH & - & O - R^2 \\ & & \vert & & & & \vert & & \\ & & R^1 & & & & Az & & \end{array} \qquad (Ia)$$

,

in der

Ar, $R^1$, $R^2$ und Az die im Vorstehenden angegebene Bedeutung haben,

mit Hilfe von Reduktionsmitteln reduziert.

4. Fungizide Mittel, enthaltend wenigstens ein 3-substituiertes Azolyl-butan-Derivat der allgemeinen Formel (I) nach Anspruch 1 und Anspruch 3.

5. Pflanzenschutzmittel, enthaltend wenigstens ein 3-substituiertes Azolyl-butan-Derivat der allgemeinen Formel (I) nach Anspruch 1 und Anspruch 3.

6. Verfahren zur Bekämpfung einer Pflanzenerkrankung, dadurch gekennzeichnet, daß ein 3-substituiertes Azolyl-butan-Derivat der allgemeinen Formel (I) nach Anspruch 1 und Anspruch 3 entweder allein oder im Gemisch mit einem Verdünnungsmittel und/oder einem oberflächenaktiven Mittel und, falls weiter erforderlich, mit einem stabilisierenden Mittel, einem Haftmittel oder einem synergistischen Mittel zur Anwendung gebracht wird.

7. 1-(4-Chlorophenoxy)-1-(1,2,4-triazol-1-yl)-3-methyl-3-phenyl-butan-2-on nach Anspruch 1, gekennzeichnet durch die Formel

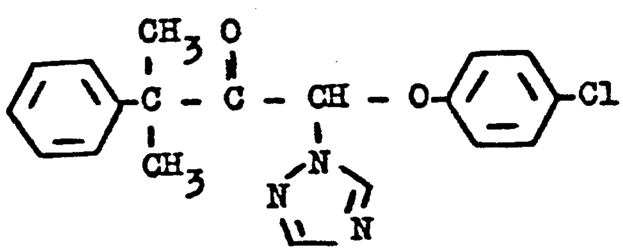

8. 1-Phenoxy-1-(1,2,4-triazol-1-yl)-3-methyl-3-(2-chlorophenyl)-butan-2-on nach Anspruch 1, gekennzeichnet durch die Formel

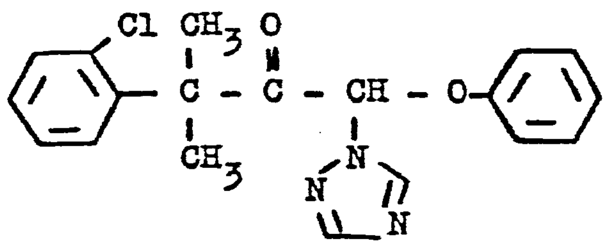

9. 1-(4-Chlorophenoxy)-1-(1,2,4-triazol-1-yl)-3-methyl-3-(3-chlorophenyl)-butan-2-on nach Anspruch 1, gekennzeichnet durch die Formel

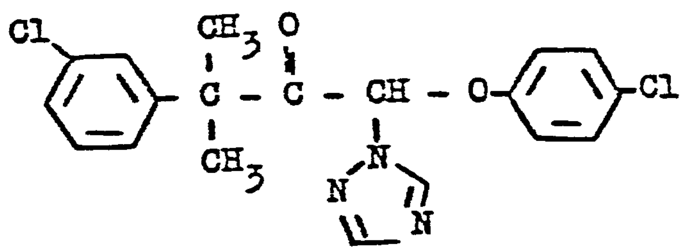

10. 1-(4-Chlorophenoxy)-1-(1-imidazolyl)-3-methyl-3-phenylbutan-2-on nach Anspruch 1, gekennzeichnet durch die Formel